(19) Europäisches Patentamt
European Patent Office
Office européen des brevets

(11) Veröffentlichungsnummer: **0 206 078 A2**

(12) **EUROPÄISCHE PATENTANMELDUNG**

(21) Anmeldenummer: **86107845.9**

(22) Anmeldetag: **09.06.86**

(51) Int. Cl.⁴: **A 01 N 43/90**, C 07 D 487/04
// (C07D487/04, 249:00, 239:00)

(30) Priorität: **22.06.85 DE 3522463**

(43) Veröffentlichungstag der Anmeldung: **30.12.86 Patentblatt 86/52**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Jelich, Klaus, Dr., Pahlkestrasse 5, D-5600 Wuppertal 1 (DE)**
Erfinder: **Santel, Hans-Joachim, Dr., Grünstrasse 9a, D-5090 Leverkusen 1 (DE)**
Erfinder: **Schmidt, Robert R., Dr., Im Waldwinkel 110, D-5060 Bergisch Gladbach 2 (DE)**

(54) **Herbizide Mittel.**

(57) Die Erfindung betrifft die Verwendung von teilweise bekannten Triazolo-pyrimidinen der Formel (I),

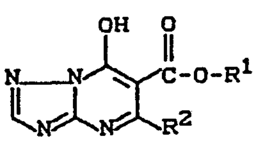

(I)

in welcher
$R^1$ für Alkyl steht und
$R^2$ für Wasserstoff oder Alkyl steht,
als Herbizide.
Die Erfindung betrifft weiterhin neue Triazolo-pyrimidine der Formel (Ia),

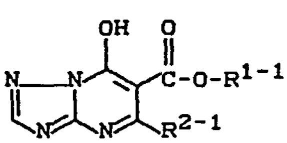

(Ia)

in welcher
$R^{1-1}$ für Alkyl steht und
$R^{2-1}$ für Wasserstoff oder Alkyl steht,
wobei jedoch nicht gleichzeitig $R^{1-1}$ für Ethyl steht, wenn $R^{2-1}$ für Wasserstoff steht.

EP 0 206 078 A2

0206078

BAYER AKTIENGESELLSCHAFT 5090 Leverkusen, Bayerwerk
Konzernverwaltung RP
Patentabteilung KM/Kü-c
IIa/Ia

Herbizide Mittel

Die Erfindung betrifft die Verwendung von teilweise bekannten Triazolo-pyrimidinen als Herbizide.

Es ist bereits bekannt, daß bestimmte Triazol-Derivate, wie beispielsweise das 3-Amino-1,2,4-triazol herbizide Eigenschaften besitzen (vergl.z.B. K.H.Büchel 'Pflanzenschutz- und Schädlingsbekämpfungsmittel', G.Thieme Verlag, Stuttgart 1977, S.181).

Deren herbizide Wirksamkeit gegenüber Problemunkräutern ist jedoch ebenso wie ihre Verträglichkeit gegenüber wichtigen Kulturpflanzen nicht immer in allen Anwendungsbereichen völlig zufriedenstellend.

Außerdem sind bestimmte Triazolo-pyrimidine, wie beispielsweise das 6-Ethoxycarbonyl-7-hydroxy-[1,2,4]-triazolo-[1,5-a]-pyrimidin bekannt(vgl.z.B. US-PS 3.190.752).Über eine herbizide Wirkung dieser Verbindung ist bisher nichts bekannt.

Es wurde gefunden, daß die teilweise bekannten Triazolo-pyrimidine der allgemeinen Formel (I),

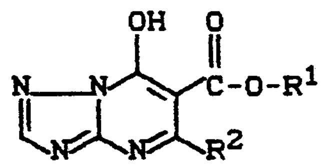

(I)

in welcher

$R^1$ für Alkyl steht und

$R^2$ für Wasserstoff oder Alkyl steht,

herbizide, insbesondere auch selektiv-herbizide Eigenschaften besitzen.

Überraschenderweise zeigen die erfindungsgemäß verwendbaren Triazolo-pyrimidine der allgemeinen Formel (I) neben einer erheblich verbesserten herbiziden Wirkung gegenüber Problemunkräutern auch eine deutlich verbesserte Verträglichkeit gegenüber wichtigen Kulturpflanzen als die aus dem Stand der Technik bekannten Triazol-Derivate, wie beispielsweise das 3-Amino-1,2,4-triazol, welche chemisch und wirkungsmäßig naheliegende Verbindungen sind.

Die erfindungsgemäß verwendbaren Triazolo-pyrimidine sind durch die Formel (I) allgemein definiert. Bevorzugt sind Verbindungen der Formel (I), bei welchen

$R^1$ für geradkettiges oder verzweigtes Alkyl mit 1 bis 8 Kohlenstoffatomen steht und

$R^2$ für Wasserstoff oder für geradkettiges oder verzweigtes Alkyl mit 1 bis 8 Kohlenstoffatomen steht.

Besonders bevorzugt verwendet man Verbindungen der Formel (I), bei welchen

$R^1$ für Methyl, Ethyl, n- oder i-Propyl, sowie n-, i-, s- oder t-Butyl steht und

$R^2$ für Wasserstoff, Methyl, Ethyl, n- oder i-Propyl, sowie n-, i-, s- oder t-Butyl steht.

Im einzelnen seien außer den bei den Herstellungsbeispielen genannten Verbindungen die folgenden Triazolo-pyrimidine der allgemeinen Formel (I) genannt:

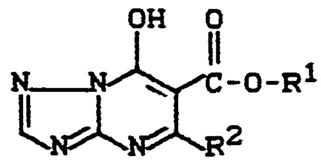

(I)

| $R^1$ | $R^2$ | $R^1$ | $R^2$ |
|---|---|---|---|
| $CH_3$ | $C_2H_5$ | $n\text{-}C_3H_7$ | $n\text{-}C_3H_7$ |
| $CH_3$ | $n\text{-}C_3H_7$ | $n\text{-}C_3H_7$ | $n\text{-}C_4H_9$ |
| $CH_3$ | $n\text{-}C_4H_9$ | $i\text{-}C_3H_7$ | H |
| $CH_3$ | $i\text{-}C_3H_7$ | $i\text{-}C_3H_7$ | $CH_3$ |
| $C_2H_5$ | $C_2H_5$ | $i\text{-}C_3H_7$ | $C_2H_5$ |
| $C_2H_5$ | $n\text{-}C_3H_7$ | $i\text{-}C_3H_7$ | $i\text{-}C_3H_7$ |
| $C_2H_5$ | $i\text{-}C_3H_7$ | $n\text{-}C_4H_9$ | H |
| $C_2H_5$ | $n\text{-}C_4H_9$ | $n\text{-}C_4H_9$ | $CH_3$ |
| $n\text{-}C_3H_7$ | $CH_3$ | $n\text{-}C_4H_9$ | $C_2H_5$ |
| $n\text{-}C_3H_7$ | $C_2H_5$ | $n\text{-}C_4H_9$ | $n\text{-}C_3H_7$ |
| | | $n\text{-}C_4H_9$ | $n\text{-}C_4H_9$ |

Die erfindungsgemäß verwendbaren Triazolopyrimidine der Formel (I) sind teilweise bekannt (vergl. z.B. US-PS 3.190.752 oder DE-OS 26 48 770).

Noch nicht bekannt sind Triazolo-pyrimidine der Formel (Ia),

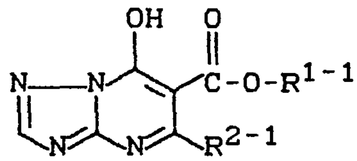

(Ia)

in welcher

$R^{1-1}$ für Alkyl steht und

$R^{2-1}$ für Wasserstoff oder Alkyl steht,

wobei jedoch nicht gleichzeitig $R^{1-1}$ für Ethyl steht, wenn $R^{2-1}$ für Wasserstoff steht.

Bevorzugt sind Verbindungen der Formel (Ia), bei welchen $R^{1-1}$ für geradkettiges oder verzweigtes Alkyl mit 1 bis 8 Kohlenstoffatomen steht und $R^{2-1}$ für Wasserstoff oder für geradkettiges oder verzweigtes Alkyl mit 1 bis 8 Kohlenstoffatomen steht, wobei jedoch nicht gleichzeitig $R^{1-1}$ für Ethyl steht, wenn $R^{2-1}$ für Wasserstoff steht.

Besonders bevorzugt sind Verbindungen der Formel (Ia). bei welchen $R^{1-1}$ für Methyl, Ethyl, n- oder i-Propyl oder n-, i-, s- oder t-Butyl steht und $R^{2-1}$ für Wasserstoff, Methyl, Ethyl, n- oder i-Propyl sowie n-, i-, s- oder t-Butyl steht, wobei jedoch nicht gleichzeitig $R^{1-1}$ für Ethyl steht, wenn $R^{2-1}$ für Wasserstoff steht.

Man erhält die noch nicht bekannten Triazolo-pyrimidine der Formel (Ia) in Analogie zu bekannten Verfahren, wenn man Aminotriazole der Formel (II),



(II)

in welcher

R für Wasserstoff oder eine Carboxylgruppe steht,

mit Malonesterderivaten der Formel (III),

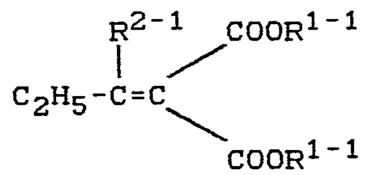

(III)

in welcher

$R^{1-1}$ und $R^{2-1}$ die oben angegebene Bedeutung haben,

gegebenenfalls in Gegenwart eines Verdünnungsmittels umsetzt.

Man erhält die bekannten Triazolo-pyrimidine der Formel (I) in Analogie zu den hier beschriebenen Herstellungsverfahren der Verbindungen der Formel (Ia) und in Analogie zu bekannten Verfahren.

Verwendet man beispielsweise 5-Amino-1,2,4-triazol-3-carbonsäure und Ethoxymethylenmalonsäuredimethylester als Ausgangsstoffe, so läßt sich der Reaktionsablauf des Herstellungsverfahrens durch das folgende Formelschema darstellen:

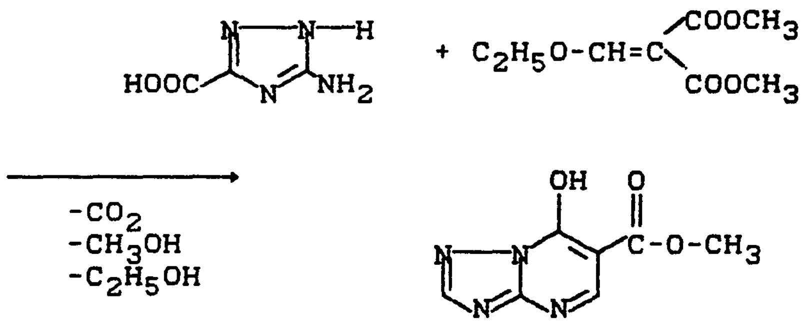


Die zur Durchführung des Herstellungsverfahrens als Ausgangsstoffe benötigten Aminotriazole sind durch die Formel (II) allgemein definiert. Die Aminotriazole der Formel (II), bei welchen R für Wasserstoff oder eine Carboxygruppe steht sind allgemein bekannte Verbindungen der organischen Chemie.

Die zur Durchführung des Herstellungsverfahrens weiterhin als Ausgangsstoffe benötigten Malonesterderivate sind durch die Formel (III) allgemein definiert. In dieser Formel (III) steht $R^{1-1}$ vorzugsweise für geradkettiges oder verzweigtes Alkyl mit 1 bis 8 Kohlenstoffatomen, insbesondere für Methyl, Ethyl, n- oder i-Propyl, sowie n-, i-, s- oder t-Butyl; $R^{2-1}$ steht vorzugsweise für Wasserstoff oder für geradkettiges oder verzweigtes Alkyl mit 1 bis 8 Kohlenstoffatomen, insbesondere für Wasserstoff, Methyl, Ethyl, n- oder i-Propyl sowie n-, i-, s- oder t-Butyl.

Die Malonesterderivate der Formel (III) sind ebenfalls allgemein bekannte Verbindungen der organischen Chemie.

Als Verdünnungsmittel zur Durchführung des Herstellungsverfahrens kommen inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Alkohole wie Methanol, Ethanol oder Propanol oder Carbonsäuren wie Essigsäure.

Die Reaktionstemperaturen können bei der Durchführung des Herstellungsverfahrens in einem größeren Bereich variiert werden. Im allgemeinen arbeitet man bei Temperaturen zwischen 20 °C und 150°C , vorzugsweise bei Temperaturen zwischen 40 °C und 120 °C.

Zur Durchführung des Herstellungsverfahrens setzt man pro Mol an Aminotriazol der Formel (II) im allgemeinen 1,0 bis 2,5 Mol, vorzugsweise 1,0 bis 1,5 Mol an Malonesterderivat der Formel (III) ein. Die Reaktionsdurchführung, Aufarbeitung und Isolierung der Reaktionsprodukte der Formel (Ia) erfolgt nach üblichen Verfahren (vgl.z.B. J.chem.Soc.Perkin Trans.I,1980, 1347-1351).

Die erfindungsgemäß verwendbaren Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäß verwendbaren Stoffe als totale oder selektive Herbizide wirken, hängt im wesentlichen von der angewendeten Menge ab.

Die erfindungsgemäß verwendbaren Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäß verwendbaren Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäß verwendbaren Wirkstoffe mit besonders gutem Erfolg zur selektiven Bekämpfung mono- und dikotyler Unkräuter, insbesondere in Maiskulturen einsetzen.

In entsprechend verringerten Aufwandmengen zeigen die erfindungsgemäß verwendbaren Wirkstoffe darüberhinaus zusätzlich einen gute fungizide Wirksamkeit und lassen sich beispielsweise zur Bekämpfung von Oomyceten einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit,

0206078

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit 2-Chlor-4-ethylamino-6-isopropylamino-1,3,5-triazin; 2-Chlor-4-ethylamino-6-(3-cyanopropylamino)-1,3,5-triazin;2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; 6-Chlor- 3-phenyl-pyridazin-4-yl-S-octyl-thiocarbonat;3,5-Diiod-4-hydroxybenzonitril; 3-Isopropyl-2,1,3-benzothiadiazin-4-on-2,2-dioxid; 2-(3,5-Dichlorphenyl)-2-(2,2,2-trichlorethyl)-oxiran; 2-Methoxy-3,6-dichlor-benzoesäure bzw. -benzoesäuremethylester oder 3,6-Dichlor-2-pyridincarbonsäure sind gegebenenfalls von Vorteil.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

0206078

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäß verwendbaren Wirkstoffe können sowohl vor als auch nach dem Auflaufen der Pflanzen appliziert werden.
Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäß verwendbaren Wirkstoffe geht aus den nachfolgenden Beispielen hervor.

<u>Herstellungsbeispiele:</u>

<u>Beispiel 1:</u>

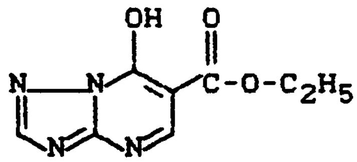

254 g (1,98 Mol) 5-Amino-1,2,4-triazol-3-carbonsäure und 472 g (2,18 Mol) Ethoxymethylenmalonsäurediethylester werden in 1000 ml Eisessig 10 Stunden unter Rückfluß erhitzt. Zur erhaltenen Reaktionsmischung gibt man unter Rühren 1500 ml Ether und saugt den so erhaltenen Feststoff ab, nimmt ihn in 600 ml Wasser auf, saugt wiederum ab und wäscht mit Ethanol und Ether nach.
Man erhält 245 g (59 % der Theorie) an 7-Hydroxy-[1,2,4]-triazolo-[1,5-a]-pyrimidin-6-carbonsäureethylester vom Schmelzpunkt 230°C.

In entsprechender Weise und gemäß den allgemeinen Angaben zur Herstellung erhält man die folgenden Triazolo-pyrimidine der allgemeinen Formel (I):

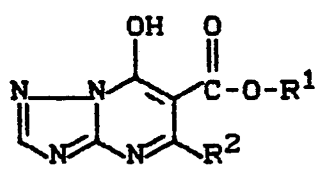
(I)

Tabelle 1

| Bsp.Nr. | $R^1$ | $R^2$ | Schmelzpunkt /° C |
|---|---|---|---|
| 2 | $CH_3$ | H | 195-200 |
| 3 | $CH_3(CH_2)_2-$ | H | 150-156 |
| 4 | $C_2H_5$ | $CH_3$ | |
| 5 | $CH_3$ | $CH_3$ | |
| 6 | $C_3H_7$-i | H | 194-201 |
| 7 | $C_4H_9$-i | H | 223 |

0206078

Anwendungsbeispiele:

In dem folgenden Anwendungsbeispiel wurde die nachstehend aufgeführte Verbindung als Vergleichsubstanz eingesetzt:

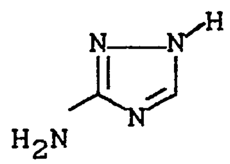

(A)

3-Amino-1,2,4-(1H)-triazol

(bekannt aus K.H. Büchel "Pflanzenschutz- und Schädlingsbekämpfungsmittel", G. Thieme Verlag, Stuttgart 1977, S. 181).

**Beispiel A**

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit, ebenso wie in der Nutzpflanzenselektivität, gegenüber dem Stand der Technik zeigt in diesem Test z.B. die Verbindung gemäß Herstellungsbeispiel: 1.

Patentansprüche

1. Herbizide Mittel, gekennzeichnet durch einen Gehalt an mindestens einem Triazolo-pyrimidin der Formel (I),

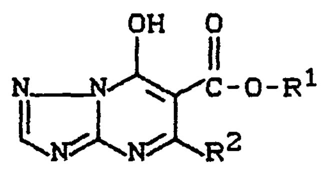

(I)

in welcher

$R^1$ für Alkyl steht und

$R^2$ für Wasserstoff oder Alkyl steht.

2. Herbizide Mittel, gekennzeichnet durch einen Gehalt an mindestens einem Triazolo-pyrimidin der Formel (I) gemäß Anspruch 1, in welcher

$R^1$ für geradkettiges oder verzweigtes Alkyl mit 1 bis 8 Kohlenstoffatomen steht und

$R^2$ für Wasserstoff oder für geradkettiges oder verzweigtes Alkyl mit 1 bis 8 Kohlenstoffatomen steht.

3. Herbizide Mittel, gekennzeichnet durch einen Gehalt an mindestens einem Triazolo-pyrimidin der Formel (I) gemäß Anspruch 1,

in welcher

$R^1$ für Methyl, Ethyl, n- oder i-Propyl oder n-, i-, s- oder t-Butyl steht und

$R^2$ für Wasserstoff, Methyl, Ethyl, n- oder i-Propyl oder n-, i-, s- oder t-Butyl steht.

4. Verfahren zur Bekämpfung von Unkraut, dadurch gekennzeichnet, daß man Triazolo-pyrimidine der Formel (I) gemäß Anspruch 1 auf Unkraut und/oder dessen Lebensraum einwirken läßt.

5. Verwendung von Triazolo-pyrimidinen der Formel (I) gemäß Anspruch 1 zur Bekämpfung von Unkraut.

6. Verfahren zur Herstellung von herbiziden Mitteln, dadurch gekennzeichnet, daß man Triazolo-pyrimidine der Formel (I) gemäß Anspruch 1 mit Streckmitteln und/oder oberflächenaktiven Mitteln vermischt.

7. Triazolo-pyrimidine der Formel (Ia)

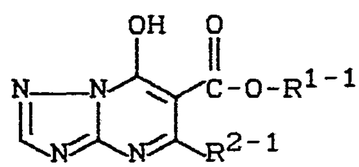

(Ia)

in welcher

$R^{1-1}$ für Alkyl steht und

$R^{2-1}$ für Wasserstoff oder Alkyl steht,

wobei jedoch nicht gleichzeitig $R^{1-1}$ für Ethyl steht, wenn $R^{2-1}$ für Wasserstoff steht.

8. Verfahren zur Herstellung von Triazolo-pyrimidinen der Formel (Ia),

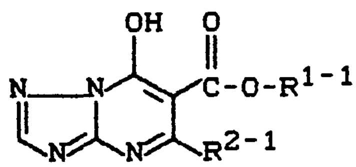

(Ia)

in welcher

$R^{1-1}$ für Alkyl steht und

$R^{2-1}$ für Wasserstoff oder Alkyl steht,

wobei jedoch nicht gleichzeitig $R^{1-1}$ für Ethyl steht, wenn $R^{2-1}$ für Wasserstoff steht,

dadurch gekennzeichnet, daß man Amino-triazole der Formel (II),

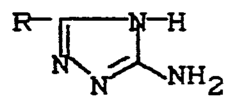

(II)

in welcher

R für Wasserstoff oder eine Carboxylgruppe steht,

mit Malonesterderivaten der Formel (III),

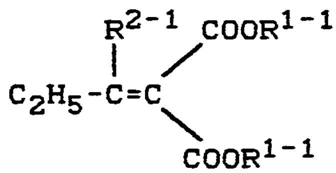

(III)

in welcher

$R^{1-1}$ und $R^{2-1}$ die oben angegebene Bedeutung haben,

gegebenenfalls in Gegenwart eines Verdünnungsmittels umsetzt.